Europäisches Patentamt

(19) European Patent Office

Office européen des brevets

(11) Publication number: **0 087 484**
**B1**

(12) **EUROPEAN PATENT SPECIFICATION**

(45) Date of publication of patent specification: **04.06.86**

(21) Application number: **82101518.7**

(22) Date of filing: **27.02.82**

(51) Int. Cl.⁴: **G 01 N 21/90**

(54) **Method of detecting foreign matters mixed in a liquid contained in transparent receptacles and apparatus relevant thereto.**

(43) Date of publication of application:
**07.09.83 Bulletin 83/36**

(45) Publication of the grant of the patent:
**04.06.86 Bulletin 86/23**

(84) Designated Contracting States:
**BE CH DE FR GB IT LI NL SE**

(56) References cited:
**US-A-4 058 737**
**US-A-4 087 184**
**US-A-4 274 745**

**L'ONDE ELECTRIQUE, vol. 59, no. 2, February 1979, pages 26-27, Paris (FR); "Fibres optiques et lentilles ( SELFOC)".**

**APPLIED OPTICS, vol. 19, no. 7, April 1980, pages 1105-1112, New York (USA); M. KAWAZU et al.: "Application of gradient-index fiber arrays to copying machines".**

(73) Proprietor: **Eisai Co., Ltd.**
**6-10, Koishikawa 4-chome Bunkyo-ku**
**Tokyo 112 (JP)**

(72) Inventor: **Takahashi, Toshio**
**No. 2677-2, Honjo-shi**
**Saitama-ken (JP)**

(74) Representative: **Liebau, Gerhard, Dipl.-Ing.**
**Birkenstrasse 39**
**D-8900 Augsburg 22 (DE)**

EP 0 087 484 B1

Courier Press, Leamington Spa, England.

# Description

The present invention relates to a method of detecting foreign matters mixed in a liquid contained in a transparent receptacle, and to an apparatus relevant thereto. To be more precise, it relates to a method of detecting undesirable, minute foreign matters that might be present within an air-tight transparent receptacle such as ampoules filled with liquid medicine, as well as an apparatus relevant thereto.

According to US—A—4 274 745 for the purpose of detecting foreign matters, there is known a method employing an apparatus which is devised as follows: transparent receptacle filled with a liquid are placed at regular intervals on the periphery of a turnable which rotates continuously as elucidated later on, and each of these receptacles are brought to a sudden stop after rotating at high speed about its own axis, whereby foreign matter mixed in the liquid swirls up and is suspended in the liquid. A beam of light is projected to each of the thus stopped receptacles at almost a right angle from of a light projection means and the light transmitted through the receptacle is detected by a light detector means which is positioned in alignment with the projection means on the opposite side of the receptacle and moves synchronously with the projector; thus, when there occurs any decrease in the intensity of the transmitted light in respect of the image formed on the light detection means due to interruption of the light by foreign matters in the course of its transmission through the liquid, it is detected and the presence of foreign matters is thereby detected.

In an apparatus of this type, the projection means is provided with a condenser lens for projecting the light beam at the receptacle, while the light detector is provided with a focusing lens for focusing that light beam transmitted through the receptacle. As a focusing lens for this purpose, a lens provided for use in ordinary cameras as lens system that displays a sufficient resolution even on minute foreign matters having a particle size of 50 μ or thereabouts has been used. In this context, said focusing lens is required to be capable of not only forming a distinct image of minute foreign matters on the light detection means but of detecting the whole flux of light emitted from a light source and transmitted through the receptacle to make the brightness of the background as high and uniform as possible in order to improve the inspecting sensitivity, and therefore it has hitherto been unavoidable to employ a focusing lens having an aperture larger than the depth of the liquid within the receptacle.

As a consequence, the prior art is defective in that, though it is possible to perform the inspection by the use of an ordinary lens set of about 40 mm in aperture having a focal distance of, for instance, f=55/F1.4 in the case of amples of 2 ml or 5 ml, it is extremely difficult to materialize a focusing lens having a sufficient resolution on minute foreign matters and an adequate aperture for use in inspection a receptacle wherein the depth of the liquid as 100 mm or more, such as 500 ml liquid supply bottle.

Besides, the prior art is defective in that, employment of a focusing lens having a large aperture necessitates provision of a considerably wide space between the focusing lens and the light receiving surface, entailing requirement for enlargement of the light detection means as a whole that is not only difficult to design but apt to cause oscillation of the apparatus at the time of reciprocating motion during the inspection and bring about blurring of the resulting image. To be more precise, in the case where a lens having a focal distance of 135 mm, for instance, is employed, in order to obtain an equimultiplied image, it is necessary to make the distance between the receptacle and the lens and that between the lens and the light-receiving surface twice longer than the foregoing focal distance, or 270 mm. Accordingly, the distance between the receptacle and the light-receiving surface is required to be 540 mm and the size of the resulting light detection means becomes large, entailing the foregoing defects.

Moreover, in the case of a transparent receptacle whose base makes a right angle with the side wall thereof, when parallel light is projected to the receptacle at almost a right angle as in the foregoing, the incident light is refracted at a large angle by the bottom of receptacle to hamper the transmission of light through the liquid within the base portion, entailing a defect that foreign matters which might be present in said portion can not be detected.

The light detection means of the known apparatus comprise a two dimensional array of microphotoreceivers which are optically connected to photoelectric elements by a bundle of optical fibers. It should be noted that the light transmitted through the receptacle is focused on to the microphotoreceivers by a focusing lens mentioned before. Thus the bundle of optical fibers is just a means for transmitting light received at the microphotoreceivers to the photoelectric elements.

US—A—4 087 184 discloses an apparatus operating, in principal, similar to that of US—A—4 274 745. In order to be able to sense contamination with a sensitivity independent of the container size and specular reflections from the liquid surface the container is virtually subdivided in to four blocks which are monitored individually. Again a focusing lens is used in order to project the light transmitted through the receptacle on to a two-dimensional array of photoelectric elements through fiber optic bundles which dissect the image in to a number of rectangular areas. The array of photo-electric elements are directed downwards at an acute angle to the horizontal.

From APPLIED OPTICS, vol. 19 No. 7, April 1980, pages 1105—1112 New York (USA), M. KAWAZU et al.: "Application of gradient-index fiber arrays to copying machines". Pages 1105—1106 and L'ONDE ELECTRIQUE, vol. 59, no. 2, February 1979, pages 26—27, Paris (FR), "Fibres optiques et lentilles (SELFOC)", it has become known to use a gradient-index fiber array as on optical system for use in copying and faximile machines. Such arrays are to replace conventional optical lenses.

It is the object of the present invention to provide a method of detecting foreign matter mixed in a liquid filled in transparent receptacles and an apparatus relevant thereto capable of testing receptacles of various sizes accurately by using a compact apparatus.

According to the present invention the foregoing object is achieved by a method according to claim 1 and an apparatus according to claim 2.

The exact nature of the present invention, as well as other objects and advantages thereof, will be readily apparent from consideration of the following specification relating to the accompanying drawings, in which like reference characters designate the same or similar parts throughout the figures thereof.

Figure 1 is an illustration for explaining the optical properties of the light-focusing glass fibers,

Figure 2 is a plan of the whole of an embodiment of the foreign matter detecting apparatus according to the present invention,

Figure 3 is an enlarged cross-section of the apparatus shown in Fig. 2 as partially cut vertically along line III—III thereof,

Figure 4 is an enlarged detail plan of a part of the illustration in Fig. 2,

Figure 5 is an enlarged cross-section of the figure shown in Fig. 4 as partially cut vertically along the line V—V thereof.

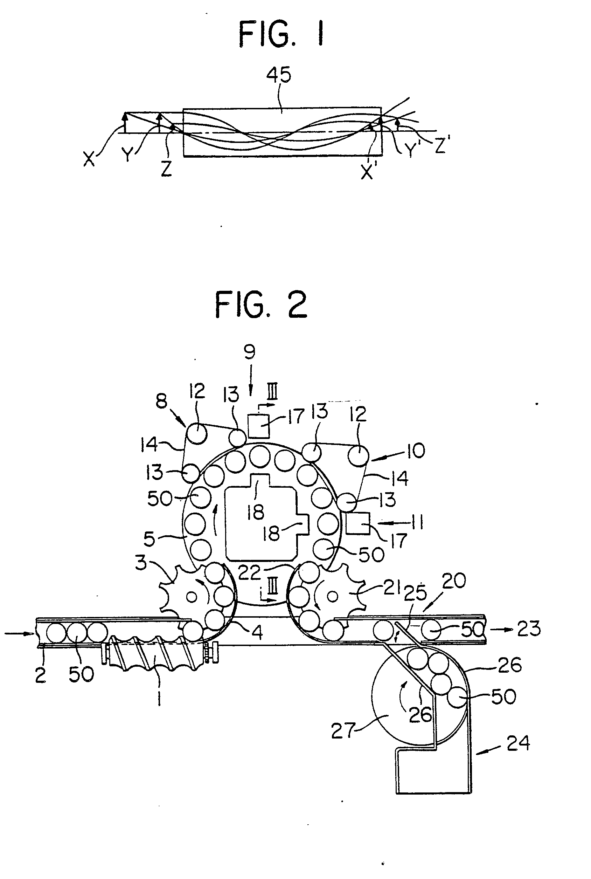

In the embodiment of the present invention illustrated in Figs. 2 through 5, parts other than the light projectors 17 and 17' and the light detectors 18 and 18' in the first inspection means 9 and the second inspection means 11 belong to well-known arts. Therefore, some of them that are of no particular importance are herein omitted from illustration, and also as to other well-known parts than the above defined, explanation thereof will be simplified.

First, the well-known parts in the present embodiment will be explained.

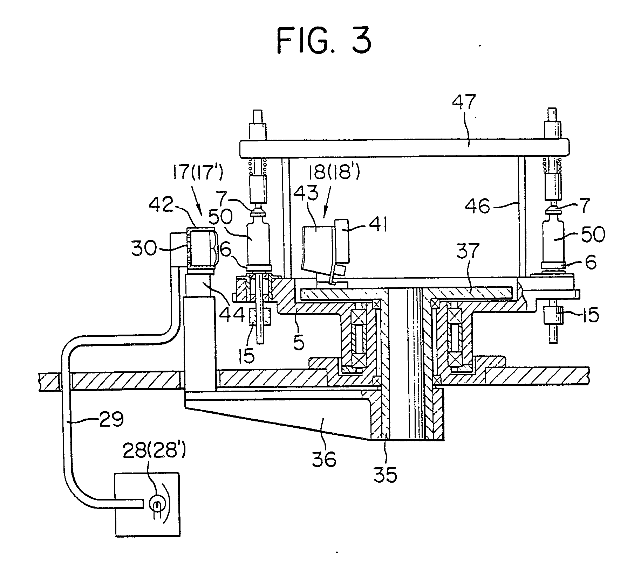

Referring to Figs. 2 and 3, the reference numeral 1 denotes a screw feeder which supplies receptacles loaded on a belt conveyor 2 to a star wheel 3. The receptacles 50 supplied to the notches of the star wheel 3 are guided by a guide plate 4 with the rotation of the star wheel 3 to be supplied to seats 6 of a turntable 5.

The turntable 5 rotates continuously and synchronously with the star wheel 3, and the receptacles 50 supplied to the seats 6 of this turntable 5 are firmly held by caps 7 which hang in vertically movable fashion from a supporting plate 47 mounted on posts 46 erected on the turntable 5. On the periphery of the turntable 5 there are installed the first receptacle turning means 8, the first inspection means 9, the second receptacle turning means 10 and the second inspection means 11, and the first and second receptacle turning means are respectively provided with a motor 12, a pulley 13 and a belt 14. This belt 14 comes in contact with a pulley 15 fixed on the supporting shaft of the seat 6 supported on the turntable 5 in rotatable fashion, and rotates the receptacles 50 at high speed.

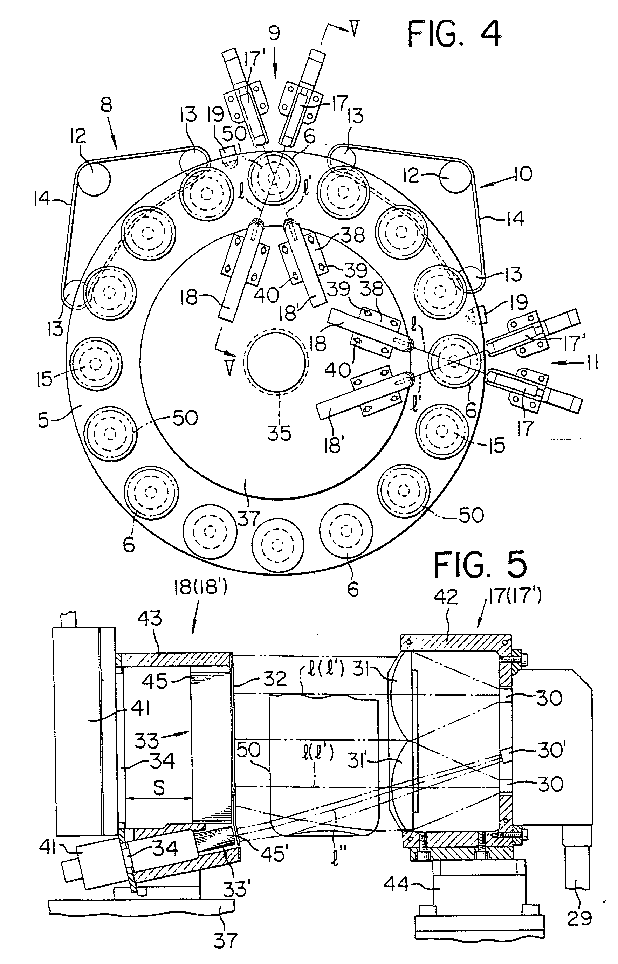

The first and second inspection means 9 and 11 are respectively equipped with a light projector 17 and a light detector 18. This light projector 17 is, as illustrated in Figs. 3 and 5, provided with a lens case 42 which accommodates condenser lenses 31, 31' disposed in the front part thereof and projection heads 30 disposed in the rear part thereof to confront the respective lenses. This lens case 42 is disposed outside the turntable 5 and mounted on a case seat 44 fixed on the upper end of an L-shaped arm 36 which projects sideways from a shaft 35 installed in rotatable fashion relative to the turntable 5. Meanwhile, the projection head 30 is connected to a lamp 28 through a light transmit means 29. The thus devised light projector 17 is disposed almost horizontally so as to be capable of projecting rays of light in almost normal direction toward the receptacle 50.

The light detector 18 is installed on a seat 38 mounted on a table 37 fixed to the upper end of a shaft 35 and is so devised that the position thereof can be freely adjusted both forward and backward by virtue of engagement of slits 39 and bolts 40 (cf. Fig. 4).

The above described light projector and light detector are disposed to be opposite to each other with a receptacle 50 supported on the turntable 5 between them.

Further, between the first receptacle turning means 8 and the first inspection means 9, as shown in Fig. 4 there is provided a brake 19 for the purpose of bringing the rotating receptacle 50 to a halt as it comes in contact with the pulley 15. Accordingly, when a receptacle held by a cap 7 disposed thereabove reaches the first receptacle turning means 8 with the rotation of the turntable 5, the belt 14 and the pulley 15 come in contact with each other and rotate at high speed, whereby the receptacle 50 rotates together with the seat 6. When the receptacle passes through the first receptacle turning means 8, the brake 19 comes in contact with the pulley 15 and brings the seat 6 to a halt. At this, the receptacle also comes to a halt, but minute foreign matters mixed in the liquid contained therein maintain suspended.

When the receptacle reaches the first inspection means 9, the table 37 moves synchronously with the turntable 5 by virtue of a reciprocating device not shown in the drawings and accordingly the light projector 17 and the light detector 18 move together with the receptacle 50, whereby inspection of the content of receptacle is performed.

This inspection has been explained in detail in Japanese Laid-Open Patent Application No.. 139597/1978. To give an epitome of the inspection hereunder, the flux of light transmitted through the receptacle 50 is focused by a focusing lens not

shown herein and is formed into an image on a bundle of optical fibers that forms a ray-receiving surface disposed in the rear of said lens. The thus formed image is then transferred by way of said bundle of optical fibers to photoelectric elements disposed in the rear thereof, and by observing increase and decrease of the luminous intensity through the output from the photoelectric element the presence of foreign matters is to be detected. When the inspection is completed in this way, the first inspection means 9 returns rapidly to this former position and performs inspection of the succeeding receptacles.

The receptacle 50 subjected to the first inspection further moves with rotation of the turntable 5 and undergoes the second inspection by the second inspection means 11 in the same way as the first inspection by way of the second receptacle turning means 10.

Based on the results of the foregoing first and second inspections, the receptacles filled with a liquid 50 are classified into qualified goods and inferior goods by a succeeding selecting means 20 (cf. Fig. 2). Transfer of said receptacles 50 from the turntable 5 to this selecting means 20 is performed by a delivery star wheel 21, whose structure is the same as that of the aforesaid star wheel 3, together with the guide plate 22.

The selecting means 20 is equipped with a damper 25 for the purpose of sifting inferior goods to be sent a delivery portion for inferior goods 23 from qualified goods to be sent to a delivery portion for qualified goods 24 with respect to the receptables 50 transported by the conveyor 2, and this damper 25 works in accordance with instructions from the inspection means 9 and 11.

The delivery portion for qualified goods 24 comprises a guide plate 26 and a turntable 27.

The foregoing processes of the conventional method of detecting foreign matters are admittedly to be followed in the present invention in almost the same manner, but the point of difference between the method proposed in the present invention and the conventional method relates to the construction of the light projector and light detector as well as the disposition thereof, so this characteristic of the present invention will be hereunder elucidated by reference to the embodiment illustrated in the accompanying drawings.

Referring to Figs. 3 through 5, the first and second inspection means 9 and 11 are respectively provided with another light projector 17' in addition to the afore described light projector 17, said light projector 17' being of the same construction, having optic axis "I" of rays of light emitted from the light projector 17 at the center of the receptacle 50 and being so disposed as to form a V-shape with the light projector 17 relative to said center of receptacle. In concert with this provision of light projector 17', another light detector 18' having the same construction as the light detector 18 for the purpose of receiving the flux of light emitted from the light projector 17' is disposed as to form a V-shape with the light detector 18 relative to the center of receptacle. The purpose in providing two sets of inspection means consisting of light projector and light detector as set forth above is to facilitate the inspection of the central portion of the receptacle 50 by means of one set consisting of, for instance, the light projector 17 and the light detector 18 and the inspection of the peripheral portion of the receptacle 50 by means of the other set consisting of the light projector 17' and the light detector 18'. In this respect further elucidation will be made later on.

As regards the light projectors 17 and 17', the construction of a portion thereof is different from that of the aforesaid conventional one. This point of difference lies in that, as seen from Fig. 5, one projection head 30 is disposed almost horizontally relative to the receptacle 50 like the conventional one, while the other projection head 30' is disposed aslant downward, preferably in such a manner as to make the optic axis "I" of rays of light pass through the lens 31' aslant downward at an angle of about 15° relative to a horizontal level. By so disposing, it becomes possible to inspect the bottom portion of the receptacle 50 in concert with the light detectors 18 and 18' described below.

Further, in the light detectors 18 and 18', in lieu of a single focusing lens employed for the conventional ray receiver, bundles 45 and 45' of gradient-index light-focusing glass fibers the fibres in each bundle being disposed parallel to one another (cf. Fig. 5) are set within a frame 32 to thereby construct focusing lenses 33 and 33', and the lens 33 is disposed face to face with the projection head 30 while the lens 33' is disposed face to face with the projection head 30'. And, at a fixed distance S in the rear of these lenses 33 and 33' there is provided a light-receiving surface 34 like in the prior art and further in the rear of this light-receiving surface there are provided photoelectric elements (not shown in the drawing) as accommodated in a casing 41.

The foregoing light-focusing glass fibers are a kind of unistructural glass fibers popular under the trade name "Selfoc", and the radial refractive index of the rays of light transmitted lengthwise is to change continuously excepting that the rays of light incident upon the optic axis are to go straight on. Accordingly, as illustrated in Fig. 1, in the case of objects "X, Y, Z" located in different positions on the optic axis, rays of light projected from these objects propagate along the optic axis to come to form an image respectively at different places along the lengthwise direction of the optic axis, such as denoted by "X', Y', Z'". Therefore, by the use of such glass fibers cut into pieces of an appropriate length, there can be provided a lens having a focal distance corresponding to said length. Also, by virtue of arranging such pieces in proportion to the length of field of vision, there can be provided a lens having a wide field of vision or a lens having a short focal distance while being capable of functioning like conventional lenses of large aperture.

In the foregoing embodiment, by virtue of utilizing the optical characteristics of said gradient-index optical fibers 45 such as described above, it also has become possible to perform inspection of the central portion and the peripheral portion of a receptacle separately as stated above.

For example, in the case of a receptacle 50 having a large diameter such as liquid supply bottles containing 200 ml, 500 ml and the like, detection of foreign matters suspended in the lower portion of the receptacle can not be performed satisfactorily by merely focusing on one point of the central portion thereof. In view of this fact, two sets of light projectors and light detectors are installed as above, and at the time of inspecting the central portion by the light detector 18 and the lower portion by the light detector 18' with respect to a receptacle containing, for instance, 500 ml of liquid, as optical fibers 45 for the former light detector 18, fibers having a length corresponding to f=50 mm are employed, while as optical fibers 45' for the latter light detector 18', fibers having a length corresponding of f=50 mm are employed. The result of detection conducted by the use of the thus devised apparatus has proved very satisfactory.

## Claims

1. A method of detecting foreign matter in a liquid contained in a transparent receptacle comprising:
— rotating said receptacle at high speed about its own axis such that the foreign matter swirls up and is suspended in the liquid;
— bringing said receptacle to a sudden stop such that the foreign matter continues to be suspended in the liquid;
— projecting a beam of light from a light projection means at said receptacle;
— detecting light transmitted through said receptacle by means of a detection means positioned in alignment with said light projection means on the opposite side of said receptacle, and
— determining the presence of foreign matter in said liquid by detecting a decrease in the intensity of the transmitted light beam characterised so that:
— the light emerging from said receptacle is focused on the light receiving surface of said detection means only by means of a bundle of gradient-index optical fibres, each of which fibres is of the type able to form an image of an object.

2. Apparatus for detecting foreign matter in a liquid contained in a transparent receptacle (50) comprising:
— means (12, 13) for rotating said receptacle at high speed about its own axis such that foreign matter swirls up and is suspended in the liquid,
— means (19) for bringing said receptacle to a sudden stop such that the foreign matter continues to be suspended in the liquid,
— projection means (17) for projecting a beam of light at said receptacle,
— detection means (34, 41) for detecting light transmitted through said receptacle, said projection means and said detection means being positioned in alignment on opposite sides of said receptacle, and
— means for detecting a decrease in the intensity of the transmitted light beam as an indication of the presence of foreign matter in said liquid, characterised by
focusing means (33, 33') comprising only a bundle of gradient-index optical fibres, each of which fibres is of the type able to form an image of an object, positioned between said receptacle and said detection means and arranged to focus light emerging from said receptacle on the light receiving surface of said detection means.

3. Apparatus as claimed in Claim 2, characterised by further projection (17'), detection (34, 41) and focusing means (33, 33') means, the respective axes of said further and first-mentioned projection, detection and focusing means being at an acute angle with respect to one another and intersecting at the vertical axis of the receptacle.

4. Apparatus as claimed in Claim 3, characterised in that said first-mentioned and further means each comprise two sets of projection and focusing means, one set having its axis arranged horizontally so as to inspect a central portion of the receptacle, while the other set has its axis tilting downwardly so as to inspect the base of the receptacle, the focal lengths of the respective focusing means (33, 33') of said two sets being different.

## Revendications

1. Procédé pour détecter des corps étrangers dans un liquide contenu dans un récipient et consistant à:
— mettre ledit récipient en rotation à grande vitesse sur son propre axe, de manière à ce que les corps étrangers tourbillonnent et soient mis en suspension dans le liquide;
— arrêter brusquement le dit récipient, de manière à ce que les corps étrangers restent en suspension dans le liquide;
— projeter un faisceau lumineux provenant d'un dispositif de projection de lumière sur ledit récipient;
— détecter la lumière transmise à travers ledit récipient au moyen d'un dispositif de détection disposé en alignement avec ledit dispositif de projection de lumière sur la face opposée du dit récipient; et
— déterminer la présence de corps étrangers dans ledit liquide, en détectant une diminution de l'intensité du faisceau lumineux transmis, caractérisé en ce que:
la lumière sortant du dit récipient est concentrée sur la surface de réception de lumière du dit dispositif de détection uniquement à l'aide d'un faisceau de fibres optiques à gradient d'indice de réfraction, chacune des dites fibres étant du type capable de former une image d'un objet.

2. Appareil pour détecter des corps étrangers dans un liquide contenu dans un récipient transparent (50) comportant:

— des dispositifs (12, 13) pour mettre en rotation ledit récipient à grande vitesse autour de son propre axe, de manière à ce que les corps étrangers tourbillonnent et soient mis en suspension dans le liquide,

— un dispositif (19) pour provoquer l'arrêt brusque du dit récipient, de manière à ce que les corps étrangers restent en suspension dans le liquide,

— un dispositif de projection (17) pour projeter un faisceau lumineux sur ledit récipient,

— un dispositif de détection (34, 41) pour détecter la lumière transmise à travers ledit récipient, ledit dispositif de projection et ledit dispositif de détection étant disposés en alignement de part et d'autre du dit récipient, et

— un dispositif pour détecter une diminution de l'intensité du faisceau lumineux transmis, servant d'indication de la présence de corps étrangers dans ledit liquide
caractérisé par

des dispositifs de concentration (33, 33') comportant uniquement un faisceau de fibres optiques à gradient d'indice de réfraction, chacune de ces fibres étant du type capable de former une image d'un objet, disposé entre ledit récipient et ledit dispositif de détection et étant conçu de manière à concentrer la lumière sortant du dit récipient sur la surface de réception de lumière du dit dispositif de détection.

3. Appareil selon la revendication 2, caractérisé par un autre dispositif de projection (17'), un autre dispositif de détection (34, 41) et un autre dispositif de concentration (33, 33'), les axes respectifs des dits dispositifs supplémentaires et des premiers dispositifs de projection, de détection et de concentration formant un angle aigu l'un par rapport à l'autre et ayant leur intersection sur l'axe vertical du récipient.

4. Appareil selon la revendication 3, caractérisé en ce que ledit dispositif mentionné en premier lieu et le dispositif supplémentaire comportent, chacun, deux systèmes de dispositifs de projection, de détection, et de concentration, un système ayant son axe disposé horizontalement de manière à contrôler une partie centrale du recipient, tandis que l'autre système a son axe incliné vers le bas, de manière à contrôler la base du récipient, les longueurs focales des dispositifs de concentration correspondants (33, 33') de ces deux systèmes étant différentes.

**Patentansprüche**

1. Verfahren zur Ermittlung von Fremdstoffen in einer in einem transparenten Behältnis enthaltenen Flüssigkeit, umfassend:

— Rotation des Behältnisses bei hoher Drehzahl um seine eigene Achse derart, daß die Fremdstoffe aufgewirbelt werden und in der Flüssigkeit schweben,

— das Behältnis zu einem plötzlichen Anhalten bringen derart, daß die Fremdstoffe noch in der Flüssigkeit schweben,

— einen Lichtstrahl von einer Lichtprojektionseinrichtung her auf das Behältnis projizieren,

— durch das Behältnis hindurchgelassenes Licht mittels einer Ermittlungseinrichtung ermitteln, die in Ausrichtung mit der Lichtprojektionseinrichtung auf der gegenüberliegenden Seite des Behältnisses angeordnet ist, und

— Ermittlung des Vorhandenseins von Fremdstoffen in der Flüssigkeit durch Ermittlung einer Abnahme bei der Intensität des durchgelassenen Lichtstrahls,
dadurch gekennzeichnet, daß

das aus dem Behältnis austretende Licht auf die lichtempfangende Oberfläche der Ermittlungsvorrichtung nur mittels eines Bündels aus optischen Gradient-Index-Fasern fokussiert wird, wobei jede der Fasern von der Art ist, die fähig ist, ein Abbild eines Objektes zu bilden.

2. Vorrichtung zur Ermittlung von Fremdstoffen in einer in einem transparenten Behältnis (50) enthaltenen Flüssigkeit, umfassend:

— eine Einrichtung (12, 13) für die Rotation des Behältnisses mit hoher Drehzahl um seine Achse derart, daß Fremdstoffe aufgewirbelt werden und in der Flüssigkeit suspendieren,

— eine Einrichtung (19), welche das Behältnis zu einem plötzlichen Anhalten bringt derart, daß die Fremdstoffe weiterhin in der Flüssigkeit schweben,

— eine Projektionseinrichtung (17) für das Projizieren eines Lichtstrahls an dem Behältnis,

— eine Ermittlungseinrichtung (34, 41) für die Ermittlung von durch das Behältnis durchgelassenes Licht, wobei die Projektionseinrichtung und die Ermittlungeinrichtung in Ausrichtung an den gegenüberliegenden Seiten des Behältnisses angeordnet sind, und

— eine Einrichtung für die Ermittlung einer Abnahme bei der Intensität des durchgelassenen Lichtstrahls als eine Anzeige des Vorhandenseins von Fremdstoffen in der Flüssigkeit,
gekennzeichnet durch

eine Fokussiereinrichtung (33, 33'), um fassend nur ein Bündel von optischen Gradient-Index-Fasern, wobei jede der Fasern von der Art ist, die fähig ist, ein Abbild eines Objektes zu bilden, zwischen dem Behältnis und der Ermittlungseinrichtung angeordnet und vorgesehen, um von dem Behältnis her austretendes Licht auf die lichtempfangende Oberfläche der Ermittlungseinrichtung zu fokussieren.

3. Vorrichtung nach Anspruch 2, gekennzeichnet durch eine weitere Projektionseinrichtung (17'), eine Ermittlungseinrichtung (34, 41) und eine Fokussiereinrichtung (33, 33'), wobei die entsprechenden Achsen der weiteren und der erstgenannten Projektions-, Ermittlungs- und Fokussiereinrichtungen unter einem spitzen Winkel zueinander angeordnet sind und sich an der vertikalen Achse des Behältnisses schneiden.

4. Vorrichtung nach Anspruch 3, dadurch gekennzeichnet, daß die erstgenannten und die weiteren Einrichtungen jeweils zwei Satz Projektions-, Ermittlungs- und Fokussiermittel aufweisen, wobei der eine Satz seine Achse horizontal angeordnet hat, so daß ein zentrales Teilstück des Behältnisses inspiziert wird, während der andere Satz seine Achse nach unten gekippt angeordnet hat, so daß die Basis des Behältnisses inspiziert wird, wobei die Brennweiten der entsprechenden Fokussiereinrichtungen (33, 33') der zwei Sätze unterschiedlich sind.

# FIG. 1

# FIG. 2

# FIG. 3

0 087 484

FIG. 4

FIG. 5

3